# EUROPÄISCHE PATENTSCHRIFT

(11) **EP 2 849 805 B1**
(45) Veröffentlichungstag und Bekanntmachung des Hinweises auf die Patenterteilung: **09.05.2018**
(21) Anmeldenummer: 13722434.1
(22) Anmeldetag: 14.05.2013
(51) Int. Cl.: A61K 51/04

(54) **SATZ UND VERFAHREN ZUR HERSTELLUNG EINES RADIOPHARMAKONS**
SET AND METHOD FOR THE PRODUCTION OF A RADIOPHARMACEUTICAL
KIT ET PROCÉDÉ DE PRODUCTION D'UN COMPOSÉ RADIOPHARMACEUTIQUE

(30) Priorität: 18.05.2012 DE 102012208376
(43) Veröffentlichungstag der Anmeldung: 25.03.2015
(73) Patentinhaber: Zentralklinik Bad Berka GmbH, 99437 Bad Berka (DE)
(72) Erfinder: MÜLLER, Dirk, 07751 Milda (DE)
(74) Vertreter: Liedtke, Markus
(86) Internationale Anmeldenummer: PCT/EP2013/059889
(87) Internationale Veröffentlichungsnummer: WO 2013/171185

(56) Entgegenhaltungen:
- WO-A1-2011/106846
- WO-A2-2006/056395
- WO-A2-2009/102378
- DIRK MUELLER ET AL: "Simplified NaCl Based 68 Ga Concentration and Labeling Procedure for Rapid Synthesis of 68 Ga Radiopharmaceuticals in High Radiochemical Purity", BIOCONJUGATE CHEMISTRY, Bd. 23, Nr. 8, 15. August 2012 (2012-08-15), Seiten 1712-1717, XP055071483, ISSN: 1043-1802, DOI: 10.1021/bc300103t
- MICHAEL K. SCHULTZ ET AL: "A new automated NaCl based robust method for routine production of gallium-68 labeled peptides", APPLIED RADIATION AND ISOTOPES, Bd. 76, 1. Juni 2013 (2013-06-01), Seiten 46-54, XP055071485, ISSN: 0969-8043, DOI: 10.1016/j.apradiso.2012.08.011
- VELIKYAN IRINA: "Positron emitting [68Ga]Ga-based imaging agents: chemistry and diversity", MEDICINAL CHEMISTRY, BENTHAM SC. PUBL, SHARIQAH (UNITED ARAB EMIRATES), Bd. 7, Nr. 5, 1. September 2011 (2011-09-01), Seiten 345-379, XP008163613, ISSN: 1875-6638, DOI: 10.2174/157340611796799195
- HD. MÜLLER, I. KLETLE, R.P. BAUM.: "Abstract P8: A New High Efficient NaO Based Cationic 68Ge/68Ga Generator Eluate 26.06.2011", THERANOSTICS- 1ST WORLD CONGRESS ON 68GA AND PEPTIDE RECEPTOR RADIONUCLIDE THERAPY, 2011,
- D. MÜLLER, I. KLETTE, R.P.BAUM: "Abstract P28: A New Synthesis Pathway and Quality Control of 68-Ga-BPAMD for Clinical Application", THERANOSTICS- 1ST WORLD CONGRESS ON 68GA AND PEPTIDE RECEPTOR RADIONUCLIDE THERAPY, 2011,
- D. MÜLLER, I. KLETTE, R.P.BAUM: "Purification and Labeling Strategies for 68Ga from 68Ge/68Ga Generator Eluate 26.06.2011", CONGRESS ON 68GA, 2011,
- FELLNER ET AL: "comparison of different phosphorous-containin ligands...", RADIOCHIM. ACTA, 99, 43-51, 2011, 2011,
- BAUM AT AL.: "Theranostics, 68Ga, and other radionuclides", RECENT REULTS IN CANCER RESEARCH (1ST WORLD CONGRESS ON 68GA AND PEPTIDE RECEPTOR RADIONUCLIDE THERAPY), 23 June 2011 (2011-06-23),
- -: "report from the 1st world congress on 68Ga", NUCLEAR MEDICINE REVIEW, 23 June 2011 (2011-06-23),

## Beschreibung

Die Erfindung betrifft einen Satz und ein Verfahren zur Herstellung eines Radiopharmakons.
Bildgebungsverfahren zur medizinischen Diagnose sind zum Teil seit Jahrzehnten gebräuchlich. Bei einigen dieser Verfahren, beispielsweise Positronen-Emissions-Spektroskopie (PET) oder Einzelphotonen-Emissionscomputertomographie (SPECT) werden Peptide, beispielsweise Edotreotid (DOTATOC) mit Radionukliden, beispielsweise ⁶⁸Gallium markiert und als Radiopharmaka, auch Tracer genannt, verwendet. Das Radiopharmakon bindet im menschlichen Körper an bestimmte Rezeptoren an, die insbesondere bei Tumorzellen überexpremiert sind. Mittels der bildgebenden Verfahren kann der erhöhte Beta-plus-Zerfall des ⁶⁸Gallium festgestellt und lokalisiert werden. Laut [I. Velikyan: Synthesis, Characterisation and Application of 68Ga-labelled Macromolecules. Dissertation, Universität Uppsala, 2005] zerfällt das Isotop ⁶⁸Gallium mit einer Halbwertszeit von 67,629 Minuten zu 89 % unter Aussendung eines Positrons mit maximal 1,9 MeV und zu 11 % unter Elektroneneinfang; jeweils in das stabile Isotop ⁶⁸Zink. Das abgegebene Positron trifft in der nuklearmedizinischen Anwendung nach wenigen Millimetern auf ein Elektron und zerstrahlt mit diesem zu zwei Photonen mit je 511 keV, wobei die beiden Photonen in einem Winkel von nahezu 180° voneinander vom Ort der Vernichtung abgestrahlt werden. Die abgestrahlten Photonen lassen sich mit entsprechenden Detektoren nachweisen und durch Rekonstruktion mehrerer Detektionsereignisse lässt sich der Ort der Annihilation recht genau bestimmen.
Aufgrund der kurzen Halbwertszeit von ⁶⁸Gallium kann das Radiopharmakon nicht längere Zeit vorgehalten werden sondern muss relativ kurze Zeit vor dem beabsichtigten Einsatz hergestellt werden.

⁶⁸Gallium wird mittels so genannter Gallium-68-Generatoren, auch ⁶⁸Ge/⁶⁸Ga-Generatoren genannt, aus ⁶⁸Germanium erzeugt. ⁶⁸Germanium hat eine Halbwertszeit von 270,8 Tagen und zerfällt in ⁶⁸Gallium. Dieses reichert sich im Generator bis zu einer durch seinen eigenen Zerfall bedingten Konzentration an. Das gebildete ⁶⁸Gallium wird mittels eines in den Generator eingeleiteten Lösungsmittels, mit dem nur Gallium, nicht jedoch Germanium eluiert, aus der stationären Phase vom Mutternuklid ⁶⁸Germanium separiert.

In bekannten Verfahren wird zum Eluieren Salzsäure mit einer Normalität von 0,05 N bis 0,4 N verwendet. Das Elutionsvolumen liegt dabei zwischen 5 ml und 10 ml. Das Eluat ist dementsprechend salzsauer und kann nicht direkt zum Markieren von Peptiden verwendet werden.

Zu diesem Problem sind verschiedene Lösungsansätze bekannt geworden.

Beim Verfahren der anionischen Konzentrierung wird das Eluat mit einem großen Volumen konzentrierter Salzsäure versetzt, das ⁶⁸Ga mittels eines Anionenaustauschers aufgefangen und anschließend mit Wasser in eine HEPES-Pufferlösung (2-(4-(2-Hydroxyethyl)-1-piperazinyl)-ethansulfonsäure) zur Markierung von zum Beispiel Peptiden eluiert. Bei diesem Verfahren ist eine anschließende Aufreinigung des Produkts, das heißt eine Abtrennung nicht erwünschter Substanzen, erforderlich. Zudem muss mit großen Mengen Salzsäure gearbeitet werden.

Bekannt ist auch die kombinierte kationische/anionische Konzentrierung, bei der zwei unterschiedliche Kartuschen zum Kationenaustausch (SCX - strong cation exchanger) und zum Anionenaustausch (SAX - strong anion exchanger) verwendet werden.

Beim Verfahren der kationischen Konzentrierung wird das ⁶⁸Gallium an einem Kationenaustauscher (SCX) festgehalten und anschließend mit einer Aceton/Salzsäurelösung eluiert. Das gewonnene Produkt enthält daher Aceton, das vor der Anwendung am menschlichen Körper mittels Destillation bei Temperaturen über 90 °C entfernt werden muss. Zum Nachweis, dass das Aceton vollständig entfernt wurde, ist eine intensive Qualitätskontrolle, beispielsweise mittels eines Gaschromatographen erforderlich.
Der Erfindung liegt die Aufgabe zu Grunde, einen Satz, auch Kit genannt, zur verbesserten Herstellung eines Radiopharmakons sowie ein entsprechendes verbessertes Verfahren anzugeben.
Die Aufgabe wird erfindungsgemäß gelöst durch einen Satz mit den Merkmalen des Anspruchs 2 und ein Verfahren mit den Merkmalen des Anspruchs 1. Vorteilhafte Ausgestaltungen der Erfindung sind Gegenstand der Unteransprüche.

Ein erfindungsgemäßer Satz zur Herstellung eines Radiopharmakons mittels des Verfahrens nach Anspruch 1 umfasst:
- eine sterile Kationen-Austausch-Kartusche (SCX-Kartusche),
- ein Reaktionsvial mit Ethylendiaminotetra(methylenphoshonosäure) (EDTMP) als Markierungsvorläufer insbesondere lyophilisierten
- ein Lösungsvial mit einem Lösungsmittel, beispielsweise steriler wässriger Lösung aus Essigsäure und Salzsäure,
- ein Elutionsvial mit steriler Kochsalz/Salzsäurelösung,
- ein Puffersalz.
Ein Vial kann auch als Ampulle oder Septumflasche bezeichnet werden.
Das Puffersalz kann beispielsweise im Reaktionsvial oder im Lösungsvial enthalten sein.
Der Inhalt des Reaktionsvials ist vorzugsweise lyophilisiert.
Im Reaktionsvial kann zusätzlich lyophilisierte Ascorbinsäure oder ein anderer geeigneter Stabilisator vorgesehen sein. Der Stabilisator verhindert den radiolytischen Abbau der markierten Substanz während der Verwendung des Radiopharmakons.

Als Puffersalz kann beispielsweise Ammoniumacetat oder Natriumacetat verwendet werden.

### Der Satz wird wie folgt verwendet:

Ein ⁶⁸Ge/⁶⁸Ga-Generator liefert das für die Markierung benötigte ⁶⁸Gallium. Der ⁶⁸Ge/⁶⁸Ga-Generator wird mittels Salzsäure, beispielsweise der Konzentration 0,1mol/l eluiert. Auf diese Weise wird ⁶⁸Gallium eluiert. Das Generator-Eluat wird der SCX-Kartusche zugeleitet. Als SCX-Kartusche kann beispielsweise eine kieselgel-basierende (silica based) Kartusche eingesetzt werden. Die SCX-Kartusche ist beispielsweise vorkonditioniert mit 1 ml Salzsäure der Konzentration 5,5 mol/l und 10 ml Wasser. Die vorzugsweise lyophilisierte Mischung im Reaktionsvial wird mit dem Lösungsmittel aus dem Lösungsvial gelöst. Die SCX-Kartusche wird dann mittels der Lösung aus dem Elutionsvial in das Reaktionsvial eluiert.

Die sich im Reaktionsvial ergebende Reaktionslösung kann optional auf 90 °C bis 100 °C erwärmt werden, beispielsweise über einen Zeitraum von 5 Minuten bis 15 Minuten, insbesondere sieben Minuten , um die Reaktion zu beschleunigen, bei der das ⁶⁸Gallium mit dem Markierungsvorläufer zur Bildung des Tracers verbindet. Ebenso kann die Reaktion auch bei Raumtemperatur erfolgen, wobei entsprechend mehr Zeit notwendig sein kann.

Die Konzentration von ungebundenem ⁶⁸Gallium ist vorzugsweise geringer als 5 %. Die radiochemische Reinheit des Tracers ist höher als 95 %. Die Reaktionsmischung enthält keine giftigen oder bedenklichen Substanzen, so dass keine anschließende Aufreinigung erforderlich ist. Nach einer optional durchgeführten sterilen Filtration beträgt die radiochemische Ausbeute etwa 82 % (n.d.c. - non decay corrected - nicht zerfallskorrigiert).

Am Ende der Reaktion kann das Radiopharmakon durch Zusatz eines sterilen Phosphatpuffers neutralisiert werden, beispielsweise 2 ml Natriumphosphat 1 mmol/ml Na⁺, 0,6 mmol/ml PO₄³⁻, pH=7,0.

Anschließend kann eine dünnschichtchromatografische Qualitätskontrolle erfolgen. Der so hergestellte Tracer kann anschließend ohne weitere Aufreinigung als Radiopharmakon verwendet werden.

Der erfindungsgemäße Satz kann zur routinemäßig verfügbaren Anwendung in der klinischen Praxis bei ⁶⁸Ga-Markierungs-Verfahren verwendet werden. Der erfindungsgemäße Satz reduziert den Umgang mit konzentrierter Salzsäure bei der Reinigungs- und Konzentrierungsprozedur des ⁶⁸Ga-Eluats. Das erzielbare Endprodukt (Tracer) steht mit hoher Reinheit und in hoher Ausbeute von etwa 80 % bis 95 % zur Verfügung. Die Verwendung von Aceton oder anderen organischen Lösungsmitteln oder Verbindungen wie 2-(4-(2-Hydroxyethyl)-1-piperazinyl)-ethansulfonsäure (HEPES) kann dadurch ebenfalls vermieden werden. Auf diese Weise entfällt gegenüber aus dem Stand der Technik bekannten Verfahren auch der Nachweis, dass das Aceton vollständig entfernt wurde, so dass keine intensive Qualitätskontrolle, beispielsweise mittels eines Gaschromatographen erforderlich ist. Auf diese Weise wird die Produktion von Sätzen ermöglicht, die auf relativ einfache Weise durch Zugeben der Lösung zur lyophilisierten Mischung von medizinischem Personal anwendbar sind, ohne dass eine aufwendige Laborausrüstung erforderlich ist.

Die erzielten Tracer sind länger stabil als aus dem Stand der Technik bekannte Tracer, so dass Multidosenpräparate für die Markierung und Untersuchung mehrerer Patienten erzeugt werden können.

In einer Ausführungsform der Erfindung enthält das Reaktionsvial eine lyophilisierte Mischung aus Natriumacetat und dem Liganden Ethylendiamintetra(methylenphosphonsäure) (EDTMP). Der so gebildete Tracer kann insbesondere zur Knochenszintigraphie verwendet werden.

Statt Natriumacetat kann prinzipiell Ammoniumacetat verwendet werden, Natriumacetat ist jedoch besser zur Lyophilisierung geeignet.

In einer Ausführungsform der Erfindung enthält das Reaktionsvial:
- maximal 10 mg, vorzugsweise maximal 1 mg EDTMP,
- 23 mg bis 40 mg, vorzugsweise 27,6 mg Puffersalz, insbesondere Natriumacetat,
- maximal 100 mg, vorzugsweise maximal 5 mg L-Ascorbinsäure

In einer Ausführungsform der Erfindung enthält das Lösungsvial:
- 1 ml bis 10 ml Wasser sowie Salzsäure und Essigsäure in einer solchen Menge, dass der pH-Wert der Lösung aus dem Inhalt des, dem Lösungsmittel aus dem Lösungsvial und der zur Elution der SCX-Kartusche verwendeten Lösung des Elutionsvials zwischen 3 und 4 liegt.

In einer Ausführungsform der Erfindung enthält das Lösungsvial:
- 1 ml bis 10 ml, vorzugsweise 1 ml bis 7 ml Wasser
- 5 µl bis 10 µl, vorzugsweise 6,73 µl konzentrierte Salzsäure
- 5 µl bis 10 µl, vorzugsweise 7 µl bis 8 µl Essigsäure

In einer Ausführungsform der Erfindung enthält das Elutionsvial 0,25 ml bis 3 ml Elutionslösung aus 5 mol/l Natriumchlorid und 5,5mol/l Salzsäure mit 13 µl bis 100 µl, vorzugsweise 25 µl 5,5mol/l Salzsäure je ml 5 mol/l Natriumchlorid. Die SCX-Kartusche wird vorzugsweise mit 0,5 ml der NaCl/HCl-Elutionslösung eluiert.

Ein erfindungsgemäßes Verfahren zur Herstellung eines Radiopharmakons, umfasst die Schritte:
- Gewinnung eines Generator-Eluats umfassend ⁶⁸Gallium aus einem ⁶⁸Ge/⁶⁸Ga-Generator mittels Salzsäure,
- Zuführen des Generator-Eluats in eine Kationen-Austausch-Kartusche, in der das ⁶⁸Gallium festgehalten wird,
- Abtrennen eines Durchlaufs des Generator-Eluats aus der Kationen-Austausch-Kartusche,
- Eluieren des ⁶⁸Gallium aus der Kationen-Austausch-Kartusche mittels einer Lösung, umfassend Kochsalz und Salzsäure und Zuführen in eine Mischung aus Ethylendiaminotetra(methylenphosphonsäure) (EDTMP) als Markierungsvorläufer und Natriumacetat.
In einer Ausführungsform kann das Verfahren mittels des erfindungsgemäßen Satzes durchgeführt werden.
Ausführungsbeispiele der Erfindung werden im Folgenden anhand von Zeichnungen näher erläutert.
Darin zeigen:
Figur 1 eine schematische Ansicht eines Satzes zur Herstellung eines Radiopharmakons, und
Figur 2 eine Anordnung zur Herstellung eines Radiopharmakons mittels des Satzes.
Einander entsprechende Teile sind in allen Figuren mit den gleichen Bezugszeichen versehen.

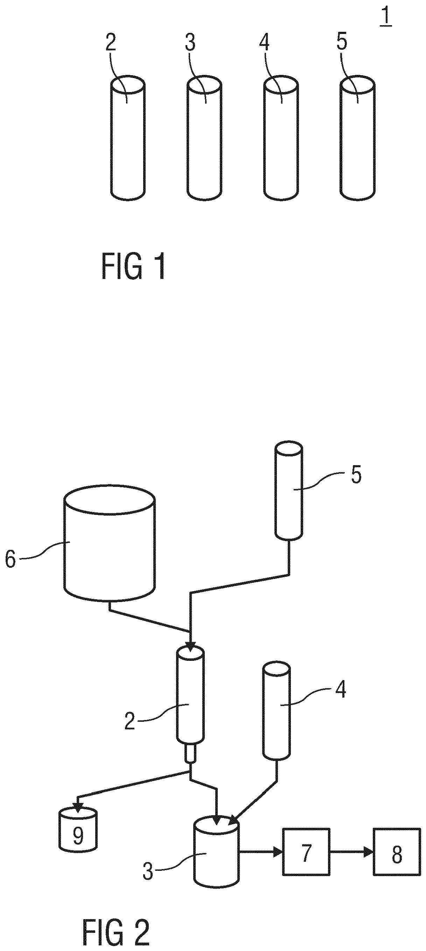

**Figur 1** zeigt eine schematische Ansicht eines Satzes 1 zur Herstellung eines Radiopharmakons. Der Satz 1 umfasst:
- eine Kationen-Austausch-Kartusche 2,
- ein Reaktionsvial 3 mit einer Mischung, umfassend einen Markierungsvorläufer und ein Puffersalz,
- ein Lösungsvial 4 mit einem Lösungsmittel,
- ein Elutionsvial 5 mit einer sterilen Lösung umfassend Kochsalz NaCl und Salzsäure HCl.

Als Markierungsvorläufer ist im Reaktionsvial 3 Ethylendiamintetra(methylen-phosphonsäure) (EDTMP) enthalten.

Die Mischung im Reaktionsvial 3 ist lyophilisiert.

Die Mischung im Reaktionsvial 3 umfasst optional Ascorbinsäure C₆H₈O₆ oder einen anderen Radikalfänger.

Das Lösungsmittel ist vorzugsweise als eine wässrige Lösung aus Essigsäure C₂H₄O₂ und Salzsäure HCl gebildet.

Als Puffersalz ist Ammoniumacetat CH₃COONH₄ oder Natriumacetat C₂H₃NaO₂ vorgesehen.

Die Kationen-Austausch-Kartusche 2 kann mit Salzsäure HCl und Wasser H₂O vorkonditioniert sein, insbesondere mit 1 ml Salzsäure HCl der Konzentration 5,5 mol/l und 10 ml Wasser H₂O.

Das Reaktionsvial 3 enthält:
- maximal 10 mg, vorzugsweise maximal 1 mg Ethylendiamintetra(methylenphosphonsäure) (EDTMP),
- 23 mg bis 40 mg, vorzugsweise 27,6 mg Puffersalz, insbesondere Natriumacetat C₂H₃NaO₂,
- maximal 100 mg, vorzugsweise maximal 5 mg L-Ascorbinsäure C₆H₈O₆

Das Lösungsvial 4 enthält:
- 1 ml bis 10 ml, vorzugsweise 1 ml bis 7 ml Wasser H₂O
- 5 µl bis 10 µl, vorzugsweise 6,73 µl konzentrierte Salzsäure HCl
- 5 µl bis 10 µl, vorzugsweise 7 µl bis 8 µl Essigsäure C₂H₄O₂.

Das Elutionsvial 5 enthält eine Menge von 0,25 ml bis 3 ml Elutionslösung aus 5 mol/l Natriumchlorid NaCl und 5,5mol/l Salzsäure HCl mit 13 µl bis 100 µl, vorzugsweise 25 µl 5,5mol/l Salzsäure HCl je ml 5 mol/l Natriumchlorid NaCl.

Der Satz 1 kann zusätzlich ein Vial mit einem Neutralisationspuffer, insbesondere einem Natriumphosphatpuffer umfassen.

**Figur 2** zeigt eine Anordnung zur Herstellung eines Radiopharmakons 8 mittels des Satzes 1.

Ein ⁶⁸Ge/⁶⁸Ga-Generator 6 liefert das für die Markierung benötigte ⁶⁸Gallium. Der ⁶⁸Ge/⁶⁸Ga-Generator 6 wird mittels Salzsäure HCl, beispielsweise der Konzentration 0,1mol/l eluiert. Auf diese Weise wird ⁶⁸Gallium eluiert und auf der Kationen-Austausch-Kartusche 2 festgehalten. Das Generator-Eluat wird der Kationen-Austausch-Kartusche 2 zugeleitet. Der Durchlauf an 0,1 mol/l HCl, gegebenenfalls mit Spuren des Mutternuklids ⁶⁸Germanium wird in einem Abfall-Sammelbehälter 9 separat aufgefangen und entsprechend den gesetzlichen Bestimmungen entsorgt. Die lyophilisierte Mischung im Reaktionsvial 3 wird mit dem Lösungsmittel aus dem Lösungsvial 4 gelöst. Die Kationen-Austausch-Kartusche 2 wird dann mittels der Lösung aus dem Elutionsvial 5 in das Reaktionsvial 3 eluiert.

Die sich im Reaktionsvial 3 ergebende Reaktionslösung kann optional auf 90 °C bis 100 °C erwärmt werden, beispielsweise über einen Zeitraum von 5 Minuten bis 15 Minuten, insbesondere sieben Minuten , um die Reaktion zu beschleunigen, bei der das ⁶⁸Gallium mit dem Markierungsvorläufer zur Bildung des Radiopharmakons 8, auch Tracer genannt, verbindet. Ebenso kann die Reaktion auch bei Raumtemperatur erfolgen, wobei sie entsprechend mehr Zeit benötigt.

Am Ende der Reaktion kann ein steriler Phosphatpuffer zugegeben werden.

Das Reaktionsprodukt kann optional mittels eines Sterilfilters 7 filtriert werden.

Der so hergestellte Tracer kann anschließend als Radiopharmakon 8 verwendet werden.

### BEZUGSZEICHENLISTE

- 1: Satz
- 2: Kationen-Austausch-Kartusche
- 3: Reaktionsvial
- 4: Lösungsvial
- 5: Elutionsvial
- 6: ⁶⁸Ge/⁶⁸Ga-Generator
- 7: Sterilfilter
- 8: Radiopharmakon
- 9: Abfall-Sammelbehälter

## Patentansprüche

1. Verfahren zur Herstellung eines Radiopharmakons (8), umfassend die Schritte:
- Gewinnung eines Generator-Eluats umfassend ⁶⁸Gallium aus einem ⁶⁸Ge/⁶⁸Ga-Generator (6) mittels Salzsäure (HCl),
- Zuführen des Generator-Eluats in eine Kationen-Austausch-Kartusche (2), in der das ⁶⁸Gallium festgehalten wird,
- Abtrennen eines Durchlaufs des Generator-Eluats aus der Kationen-Austausch-Kartusche (2),
- Eluieren des ⁶⁸Gallium aus der Kationen-Austausch-Kartusche (2) mittels einer Lösung, umfassend Kochsalz (NaCl) und Salzsäure (HCl) und Zuführen in eine Mischung aus Ethylendiamintetra(methylenphosphonsäure) (EDTMP) als Markierungsvorläufer und Natriumacetat (C₂H₃NaO₂).

2. Satz (1) zur Herstellung eines Radiopharmakons (8) mittels des Verfahrens nach Anspruch 1, wobei der Satz (1) umfasst:
- eine Kationen-Austausch-Kartusche (2),
- ein Reaktionsvial (3) mit Ethylendiamintetra(methylenphosphonsäure) (EDTMP) als Markierungsvorläufer,
- ein Lösungsvial (4) mit einem Lösungsmittel gebildet aus einer wässrigen Lösung aus Essigsäure (C₂H₄O₂) und Salzsäure (HCl),
- ein Elutionsvial (5) mit einer sterilen Lösung umfassend Kochsalz (NaCl) und Salzsäure (HCl),
- ein Puffersalz.

3. Satz (1) nach Anspruch 2, **dadurch gekennzeichnet, dass** das Puffersalz im Reaktionsvial (3) oder im Lösungsvial (4) enthalten ist.

4. Satz (1) nach einem der Ansprüche 2 oder 3, **dadurch gekennzeichnet, dass** der Inhalt des Reaktionsvials (3) lyophilisiert ist.

5. Satz (1) nach einem der Ansprüche 2 bis 4, **dadurch gekennzeichnet, dass** im Reaktionsvial (3) Ascorbinsäure (C₆H₈O₆) enthalten ist.

6. Satz (1) nach einem der Ansprüche 2 bis 5, **dadurch gekennzeichnet, dass** als Puffersalz Ammoniumacetat (CH₃COONH₄) oder Natriumacetat (C₂H₃NaO₂) vorgesehen ist.

7. Satz (1) nach einem der Ansprüche 2 bis 6, **dadurch gekennzeichnet, dass** das Reaktionsvial (3) enthält:
- maximal 10 mg, vorzugsweise maximal 1 mg Ethylendiamintetra(methylenphosphonsäure) (EDTMP),
- 23 mg bis 40 mg, vorzugsweise 27,6 mg Puffersalz, insbesondere Natriumacetat (C₂H₃NaO₂),
- maximal 100 mg, vorzugsweise maximal 5 mg L-Ascorbinsäure (C₆H₈O₆)

8. Satz (1) nach einem der Ansprüche 2 bis 7, **dadurch gekennzeichnet, dass** das Lösungsvial (4) enthält:
- 1 ml bis 10 ml, vorzugsweise 1 ml bis 7 ml Wasser (H₂O)
- 5 µl bis 10 µl, vorzugsweise 6,73 µl konzentrierte Salzsäure (HCl)
- 5 µl bis 10 µl, vorzugsweise 7 µl bis 8 µl Essigsäure (C₂H₄O₂).

9. Satz (1) nach einem der Ansprüche 2 bis 8, **dadurch gekennzeichnet, dass** das Elutionsvial (5) 0,25 ml bis 3 ml Elutionslösung aus 5 mol/l Natriumchlorid (NaCl) und 5,5mol/l Salzsäure (HCl) mit 13 µl bis 100 µl, vorzugsweise 25 µl 5,5mol/l Salzsäure (HCl) je ml 5M Natriumchlorid (NaCl) enthält.

10. Satz (1) nach einem der Ansprüche 2 bis 9, weiter umfassend ein Vial mit einem Neutralisationspuffer, insbesondere einem Natriumphosphatpuffer.

## Claims

1. A method for producing a radiopharmaceutical (8), comprising the following steps:
- obtaining a generator eluate comprising ⁶⁸gallium from a ⁶⁸Ge/⁶⁸Ga generator (6) by means of hydrochloric acid (HCl),
- passing the generator eluate into a cation exchange cartridge (2) in which the ⁶⁸gallium is held,
- removing an effluent of the generator eluate from the cation exchange cartridge (2),
- eluting the ⁶⁸gallium from the cation exchange cartridge (2) by means of a solution comprising sodium chloride (NaCl) and hydrochloric acid (HCl) and passing it into a mixture of ethylene-diaminetetra(methylenephosphonic acid) (EDTMP) as labeling precursor and sodium acetate (C₂H₃NaO₂).

2. A kit (1) for producing a radiopharmaceutical (8) according to the method of claim 1, whereby the kit comprises:
- a cation exchange cartridge (2),
- a reaction vial (3) with ethylenediaminetetra(methylenephosphonic acid) (EDTMP) as labeling precursor,
- a solution vial (4) with a solvent formed from an aqueous solution of acetic acid (C₂H₄O₂) and hydrochloric acid (HCl),
- an elution vial (5) with a sterile solution comprising sodium chloride (NaCl) and hydrochloric acid (HCl),
- a buffer salt.

3. The kit (1) as claimed in claim 2, **characterized in that** the buffer salt is present in the reaction vial (3) or in the solution vial (4).

4. The kit (1) as claimed in either of claims 2 or 3, **characterized in that** the contents of the reaction vial (3) have been lyophilized.

5. The kit (1) as claimed in any of claims 2 to 4, **characterized in that** ascorbic acid (C₆H₈O₆) is present in the reaction vial (3).

6. The kit (1) as claimed in any of claims 2 to 5, **characterized in that** ammonium acetate (CH₃COONH₄) or sodium acetate (C₂H₃NaO₂) is provided as buffer salt.

7. The kit (1) as claimed in any of claims 2 to 6, **characterized in that** the reaction vial (3) contains:
- at most 10 mg, preferably at most 1 mg of ethylene-diaminetetra(methylenephosphonic acid) (EDTMP),
- 23 mg to 40 mg, preferably 27.6 mg, of buffer salt, more particularly sodium acetate (C₃H₃NaO₂),
- at most 100 mg, preferably at most 5 mg, of L-ascorbic acid (C₆H₈O₆).

8. The kit (1) as claimed in any of claims 2 to 7, **characterized in that** the solution vial (4) contains:
- 1 ml to 10 ml, preferably 1 ml to 7 ml, of water (H₂O)
- 5 µl to 10 µl, preferably 6.73 µl, of concentrated hydrochloric acid (HCl)
- 5 µl to 10 µl, preferably 7 µl to 8 µl, of acetic acid (C₂H₄O₂).

9. The kit (1) as claimed in any of claims 2 to 8, **characterized in that** the elution vial (5) contains 0.25 ml to 3 ml of elution solution composed of 5 mol/l sodium chloride (NaCl) and 5.5 mol/l hydrochloric acid (HCl) with 13 µl to 100 µl, preferably 25 µl, of 5.5 mol/l hydrochloric acid (HCl) per ml of 5M sodium chloride (NaCl).

10. The kit (1) as claimed in any of claims 2 to 9, further comprising a vial with a neutralizing buffer, more particularly a sodium phosphate buffer.

## Revendications

1. Procédé de fabrication d'un produit radiopharmaceutique (8), comprenant les étapes suivantes :
- l'obtention d'un éluat de générateur comprenant du ⁶⁸gallium à partir d'un générateur ⁶⁸Ge/⁶⁸Ga (6) au moyen d'acide chlorhydrique (HCl),
- l'introduction de l'éluat de générateur dans une cartouche d'échange de cations (2), dans laquelle le ⁶⁸gallium est retenu,
- la séparation d'un passage de l'éluat de générateur de la cartouche d'échange de cations (2),
- l'élution du ⁶⁸gallium de la cartouche d'échange de cations (2) au moyen d'une solution comprenant du sel de cuisine (NaCl) et de l'acide chlorhydrique (HCl), et l'introduction dans un mélange d'acide éthylène-diamine-tétra(méthylène-phosphonique) (EDTMP) en tant que précurseur de marquage et d'acétate de sodium (C₂H₃NaO₂).

2. Kit (1) pour la fabrication d'un produit radiopharmaceutique (8) au moyen du procédé selon la revendication 1, dans lequel le kit (1) comprend :
- une cartouche d'échange de cations (2),
- une fiole de réaction (3) contenant de l'acide éthylène-diamine-tétra(méthylène-phosphonique) (EDTMP) en tant que précurseur de marquage,
- une fiole de solution (4) contenant un solvant formé par une solution aqueuse d'acide acétique (C₂H₄O₂) et d'acide chlorhydrique (HCl),
- une fiole d'élution (5) contenant une solution stérile comprenant du sel de cuisine (NaCl) et de l'acide chlorhydrique (HCl),
- un sel tampon.

3. Kit (1) selon la revendication 2, **caractérisé en ce que** le sel tampon est contenu dans la fiole de réaction (3) ou dans la fiole de solution (4).

4. Kit (1) selon l'une quelconque des revendications 2 ou 3, **caractérisé en ce que** le contenu de la fiole de réaction (3) est lyophilisé.

5. Kit (1) selon l'une quelconque des revendications 2 à 4, **caractérisé en ce que** de l'acide ascorbique (C₆H₈O₆) est contenu dans la fiole de réaction (3).

6. Kit (1) selon l'une quelconque des revendications 2 à 5, **caractérisé en ce que** de l'acétate d'ammonium (CH₃COONH₄) ou de l'acétate de sodium (C₂H₃NaO₂) est prévu en tant que sel tampon.

7. Kit (1) selon l'une quelconque des revendications 2 à 6, **caractérisé en ce que** la fiole de réaction (3) contient :
- au plus 10 mg, de préférence au plus 1 mg, d'acide éthylène-diamine-tétra(méthylène-phosphonique) (EDTMP),
- 23 mg à 40 mg, de préférence 27,6 mg, de sel tampon, notamment d'acétate de sodium (C₂H₃NaO₂),
- au plus 100 mg, de préférence au plus 5 mg, d'acide L-ascorbique (C₆H₈O₆).

8. Kit (1) selon l'une quelconque des revendications 2 à 7, **caractérisé en ce que** la fiole de solution (4) contient :
- 1 ml à 10 ml, de préférence 1 ml à 7 ml, d'eau (H₂O),
- 5 µl à 10 µl, de préférence 6,73 µl, d'acide chlorhydrique concentré (HCl),
- 5 µl à 10 µl, de préférence 7 µl à 8 µl, d'acide acétique (C₂H₄O₂).

9. Kit (1) selon l'une quelconque des revendications 2 à 8, **caractérisé en ce que** la fiole d'élution (5) contient 0,25 ml à 3 ml de solution d'élution constituée par du chlorure de sodium (NaCl) à 5 mol/l et de l'acide chlorhydrique (HCl) à 5,5 mol/l, avec 13 µl à 100 µl, de préférence 25 µl, d'acide chlorhydrique (HCl) à 5,5 mol/l par ml de chlorure de sodium (NaCl) à 5 M.

10. Kit (1) selon l'une quelconque des revendications 2 à 9, comprenant en outre une fiole contenant un tampon de neutralisation, notamment un tampon de phosphate de sodium.
